(19) Europäisches Patentamt
European Patent Office
Office européen des brevets

(11) **EP 4 004 925 B1**

(12) **EUROPEAN PATENT SPECIFICATION**

(45) Date of publication and mention
of the grant of the patent:
**25.06.2025 Bulletin 2025/26**

(21) Application number: **20742760.0**

(22) Date of filing: **24.07.2020**

(51) International Patent Classification (IPC):
*G16B 15/30* (2019.01)   *G16B 15/20* (2019.01)
*G16B 20/50* (2019.01)   *C07K 14/705* (2006.01)
*C07K 14/72* (2006.01)

(52) Cooperative Patent Classification (CPC):
**C07K 14/723; C07K 14/705; G16B 15/20;
G16B 15/30; G16B 20/50**

(86) International application number:
**PCT/EP2020/070907**

(87) International publication number:
**WO 2021/018756 (04.02.2021 Gazette 2021/05)**

(54) **METHOD FOR GENERATING VARIANTS OF A PROTEIN**

VERFAHREN ZUR ERZEUGUNG VON VARIANTEN EINES PROTEINS

PROCÉDÉ DE GÉNÉRATION DE VARIANTES D'UNE PROTÉINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2019 EP 19189259
02.06.2020 EP 20177770**

(43) Date of publication of application:
**01.06.2022 Bulletin 2022/22**

(73) Proprietor: **ECOLE POLYTECHNIQUE FEDERALE
DE LAUSANNE
(EPFL)
1015 Lausanne (CH)**

(72) Inventor: **BARTH, Patrick
1025 St-Sulpice (CH)**

(74) Representative: **KATZAROV S.A.
Geneva Business Center
12 Avenue des Morgines
1213 Petit-Lancy (CH)**

(56) References cited:
WO-A2-02/18590           WO-A2-2010/149964
WO-A2-2014/198528        US-A1- 2011 053 261
US-A1- 2019 147 985

- **WHITE K L ET AL: "Structural Connection
  between Activation Microswitch and Allosteric
  Sodium Site in GPCR Signaling", STRUCTURE,
  vol. 26, no. 2, 1 February 2018 (2018-02-01),
  AMSTERDAM, NL, pages 259 - 269.e5,
  XP055663000, ISSN: 0969-2126, DOI: 10.1016/
  j.str.2017.12.013**
- **MAJESKE N ET AL: "Elucidating Which Pairwise
  Mutations Affect Protein Stability: An Exhaustive
  Big Data Approach", 2017 IEEE 41ST ANNUAL
  COMPUTER SOFTWARE AND APPLICATIONS
  CONFERENCE (COMPSAC), IEEE, vol. 1, 23 July
  2018 (2018-07-23), pages 508 - 515,
  XP033409512, ISSN: 0730-3157, ISBN:
  978-1-5386-2667-2, [retrieved on 20180608], DOI:
  10.1109/COMPSAC.2018.00078**

Remarks:
The complete document including Reference
Table(s) and the Sequence Listing(s) can be
downloaded from the EPO website

## Description

### Technical Field

The present invention relates to a method for generating variants of a protein. The invention also concern a computer implemented program to carry out said method. The invention further concerns a variant of a protein obtained by the method according to the present invention and polynucleotide encoding said variant.

### Background of the art

It is known that membrane receptors sense extracellular stimuli and transduce these signals into intracellular signaling responses. Subtle differences in protein sequence often give rise to profound changes in signaling response with no obvious connection to their structure.

Allostery is a fundamental property that enables long-range communication between distant sites on a molecule. Allostery is known either for protein membrane receptor or soluble protein. It is at the origin of a large diversity of regulatory mechanisms of biological molecular functions but its biophysical underpinnings remain poorly understood. Allosteric communications are thought to be primarily mediated by intraprotein networks of coupled residues (i.e. allosteric residues) physically connecting extracellular and intracellular regions of the protein. Long-range structural coupling can promote the effective communication between distant protein sites through the propagation of even small changes in local structure and dynamics.

These observations suggest that the residue couplings that regulate the intracellular responses to extracellular stimuli largely depend on amino-acid sequence details and fine protein structure and dynamic properties. Hence, predicting how protein sequences and structures encode specific allosteric responses remains particularly challenging.

Better understanding and engineering allostery in protein would greatly benefit not only the study of protein structure, function, and pharmacology but also the design of novel biosensing protein for synthetic and cell biology applications, for instance membrane receptors.

Existing methodologies structural biologists have developed are based on empirical approaches for screening stabilized mutants to facilitate structure determination. However, studying stabilized mutants, for instance thermostabilized mutants, present serious drawback notably because they frequently fail to exhibit the ligand induced signal transduction response associated with the wild type protein.

The document WO02/18590 describes a method for identifying constitutively activating mutations where libraries of mutations are generated and screened using cell-based assays for modified receptor activities. Briefly, this is a scanning mutagenesis approach where small residues are mutated to larger ones with the hope that it will destabilize the resting state of the receptor and eventually stabilize the active state. However, the disclosed method is not a computational approach that can predict with the effect of the mutations on the receptor stability, constitutive activity and ligand-induced activity. The disclosed screening approach lacks a strong rationale for selecting the mutations, has no predictive power and rely on screening enough mutations to identify ones with desired activities. Additionally, such approach often generates mutants that behave rather poorly because mutations often destabilize the resting state instead of stabilizing the active state. An additional weakness is that a screening approach for activating mutations that is not based on a mechanistic understanding of receptor complex signaling functions may lead to receptor variants with constitutive signaling properties distinct from that of the ligand-induced WT because the mutation may inadvertently stabilize a specific subset of the active conformations accessible to the receptor in the case of GPCRs for example that are not 2-state systems.

The document WO2010/149964 describes a method for identifying stabilizing mutations of GPCRs. Based on previous findings, they identify that a local region around position 2.46 is prone to stabilization by mutagenesis. However, this approach lacks a strong rationale for selecting the mutations, has no predictive power and relies on screening enough mutations and neighboring sites to identify ones with enhanced stabilities. For instance, the resulting effects are not consistent across receptors since they vary from substantial stabilization of agonist bound form to non significant stabilization of antagonist-bound forms. Additionally, the engineered receptors tested for signaling were shown to be considerably inactivated by the mutations. Therefore, since such engineered receptors are mostly inactive, they may bias and mislead the search for new drugs.

Therefore, there is a need for a methodology to facilitate protein variant engineering and studying.

### Description of the Figures

Fig. 1 shows the *in vitro* measurements of T205I D2 activation. top. constitutive (time course); bottom. Agonist induced (dose response) as percent of maximal dopamine induced D2 WT activity. ** two-sided unpaired t-test: p < 0.05.)

Fig. 2 shows the *in vitro* measurements of T205M D2 activation. top. constitutive (time course); bottom. Agonist induced (dose response) as percent of maximal dopamine induced D2 WT activity. ** two-sided unpaired t-test: p < 0.05.)

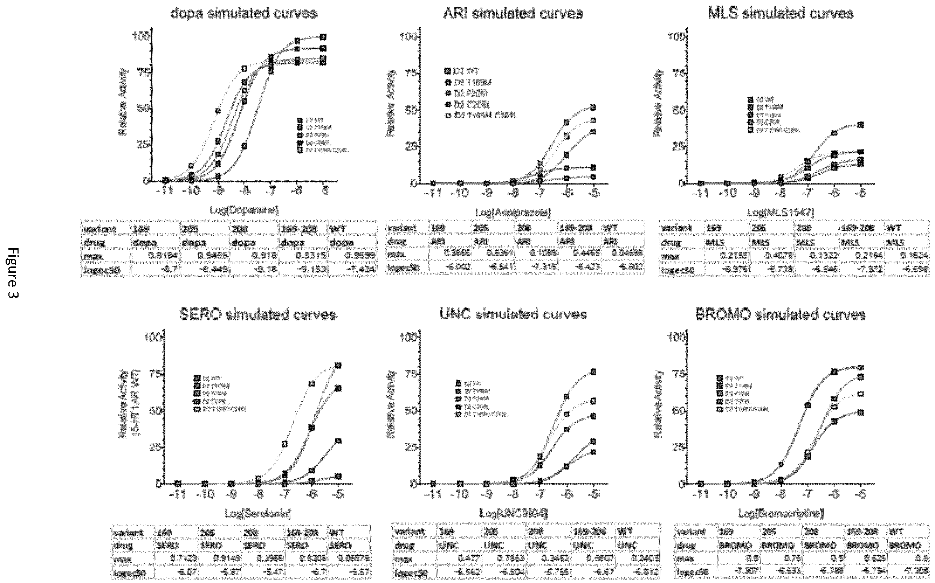

Fig. 3 shows that the mutations increase sensitivity of the proteins of the invention (T169M, F205I, C208L, T169M-C208L) to most ligands (dopamine, aripiprazole (ARI), UNC9994 (UNC), MLS1547 (MLS), serotonin).

## Summary of the Invention

The present invention concerns a computer implemented method for generating variants of a protein based on a native protein regulated by allosteric pathway, the method comprising:

- i) providing 3D structures of the native protein, said 3D structures comprising protein active conformation and inactive conformation for both ligand free and ligand bound states;
- ii) identifying at least one pair of coupled allosteric sites within the amino acid sequence of the native protein, each allosteric site being named microswitch, each microswitch consisting in one amino acid involved in regulating a signal transduced by the native protein,
- iii) generating *in silico* mutations of said identified microswitch to generate a pool of variants whose amino acid sequences comprise at least one mutation compared to the native sequence,
- iv) computing at least one score reflecting

  - iv)a) a variation in allosteric coupling for each variant compared to the allosteric coupling of the native protein, said variation being named ΔG-coupling; and/or
  - iv)b) a variation in the relative stability of each conformation for each variant, said variation being named ΔG-stability;

- v) predicting the activity of each variant compared to the native protein based on the computed score ΔG-coupling or ΔG-stability, namely

  - v)a) a change in ligand-induced activity of the variant compared to the native protein named ΔL activity, said change being calculated by

$$\Delta L \text{ activity} = (\Delta G \text{ coupling})AL - (\Delta G \text{ coupling})IL;$$

    and/or

  - v)b) a change in ligand free activity of the variant compared to the native protein named ΔC activity, said change being calculated by

$$\Delta C \text{ activity} = (\Delta G \text{ stability})A - (\Delta G \text{ stability})I$$

    wherein for the ligand free state conformations, variant protein active and inactive conformations are denoted respectively A and I;
    whereas for the ligand bound state conformations, variant protein active and inactive conformations are denoted respectively AL and IL;
    and wherein the prediction of the activity of the variant is based on a fitness function defined by:

$$F_{variant} = \Delta L \text{ activity (stability)} + \Delta C \text{ activity (coupling)} \P$$

- vi) *in vitro* testing the predicted activity of a selected variant in a validation step;
  wherein the method is arranged for generating a variant of protein with an improved parameter for the ligand compared to the one of said ligand with the native protein, said parameter being chosen among selectivity, specificity, affinity, preferably sensitivity.

Also provided is a computer implemented program comprising instructions which, when the program is executed by a computer, cause the computer to carry out the method of the invention.

**Description of the Invention**

The above problems are solved at least partially by the present invention.

The invention concerns a computer implemented method for generating variants of a protein based on a native protein regulated by allosteric pathway, the method comprising:

- i) providing 3D structures of the native protein, said 3D structures comprising protein active conformation and inactive conformation for both ligand free and ligand bound states;
- ii) identifying at least one pair of coupled allosteric sites within the amino acid sequence of the native protein, each allosteric site being named microswitch, each microswitch consisting in one amino acid involved in regulating a signal transduced by the native protein,
- iii) generating *in silico* mutations of said identified microswitch to generate a pool of variants whose amino acid sequences comprise at least one mutation compared to the native sequence,
- iv) computing at least one score reflecting

  - iv)a) a variation in allosteric coupling for each variant compared to the allosteric coupling of the native protein, said variation being named ΔG-coupling; and/or
  - iv)b) a variation in the relative stability of each conformation for each variant, said variation being named ΔG-stability;

- v) predicting the activity of each variant compared to the native protein based on the computed score ΔG-coupling or ΔG-stability, namely

  - v)a) a change in ligand-induced activity of the variant compared to the native protein named ΔL activity, said change being calculated by

$$\Delta L \text{ activity} = (\Delta G \text{ coupling})AL - (\Delta G \text{ coupling})IL;$$

    and/or

  - v)b) a change in ligand free activity of the variant compared to the native protein named ΔC activity, said change being calculated by

$$\Delta C \text{ activity} = (\Delta G \text{ stability})A - (\Delta G \text{ stability})I$$

    wherein for the ligand free state conformations, variant protein active and inactive conformations are denoted respectively A and I;
    whereas for the ligand bound state conformations, variant protein active and inactive conformations are denoted respectively AL and IL;
    and wherein the prediction of the activity of the variant is based on a fitness function defined by:

$$F_{variant} = \Delta L \text{ activity (stability)} + \Delta C \text{ activity (coupling)}$$

- vi) *in vitro* testing the predicted activity of a selected variant in a validation step;
  wherein the method is arranged for generating a variant of protein with an improved parameter for the ligand compared to the one of said ligand with the native protein, said parameter being chosen among selectivity, specificity, affinity, preferably sensitivity.

A protein regulated by allosteric pathways, for instance a membrane receptor or a soluble protein, can adopt either an active conformation or an inactive conformation for both ligand free and ligand bound states. The switch between active and inactive conformation involves smaller scale movements of individual amino acid named herein as microswitch.

A microswitch corresponds to one amino-acid at one specific position. Since, there are 20 possible amino-acids, the present invention can select 20 different possible microswitches at a given site.

Additionally, multiple microswitches at several sites can be designed simultaneously. Alternatively, the present invention also concerns combinations of microswitches comprising several single amino acids.

Advantageously, one microswitch is coupled to another microswitch pairwise. Alternatively, one microswitch can be paired with more than one microswitch.

The present invention allows generating variants of a protein by selective mutation of microswitches, preferably of at least one pair of coupled microswitch.

One aim of the invention is to select variant with microswitch that shifts the stability or the structural coupling of specific protein conformations.

For example, variants with increased constitutive activity, i.e. activity in ligand free state, namely stability microswitch, can be engineered by introducing the appropriate mutation of the identified microswitch providing an increased stability to the engineered variant. The point is to make more stabilizing contacts in the active versus the inactive ligand-free conformations. Advantageously, point amino acid mutation on stability microswitch allows to modulate the fraction of time the protein spends in each state.

Alternatively, variants with enhanced signaling response to ligand, namely allosteric microswitch, preferably agonist ligand, can be engineered by introducing the appropriate mutation of the identified microswitch to increase ligand/protein binding and/or signal transduction, for instance ligand/receptor. Advantageously, point amino acid mutation allows to modulate selectively the responses to ligands by enhancing or decreasing the protein allosteric sensing properties.

For allosteric microswitch, a score named $\Delta$G-coupling is computed. In one embodiment, $\Delta$G-coupling is calculated from the dynamics correlation between identified microswitch using an elastic model of the protein, for instance

$$RTlog\ (\alpha_x\ /\ \alpha_y) = (G^C_{A,Lb} - G^C_{I,Lb})_x - (G^C_{A,Lb} - G^C_{I,Lb})_y = \Delta G^C_x - \Delta G^C_y = \Delta\Delta G$$

With each $G^C$ = Sum (i=1; j>i; i, j<= total nb of microswitches) (Correl_dyn (i, j)). Correl_dyn is the dynamic correlation between microswitches i and j calculated using an elastic network model of the protein.

A change in ligand-induced activity of the variant compared to the native protein is determined by comparison between the $\Delta$L coupling of the variant compared to the one of the native protein. The $\Delta$L activity of the variant is calculated by

$$\Delta L\ activity = (\Delta G\ coupling)AL - (\Delta G\ coupling)IL$$

wherein variant protein active and inactive conformations are denoted respectively AL, IL for ligand bound states.

For allosteric microswitch, if $\Delta$L activity of the variant is superior to $\Delta$L activity of the native protein, it means the variant exhibits an increased sensitivity to ligand binding compared to the native protein. If $\Delta$L activity of the variant is inferior to $\Delta$L activity of the native protein, it means the variant exhibits a decreased sensitivity to ligand binding compared to the native protein.

For stability microswitch, a score named $\Delta$G-stability is computed. $\Delta$G-stability is computed by the sum of all interactions between the residues of the protein in a specific conformation and in absence of ligand (i.e. the total free energy of the system). In other words, the $\Delta$G-stability is computed from the free energy difference between each conformation in the absence of ligand.

A change in ligand-free activity of the variant compared to the native protein is determined by comparison between the $\Delta$L stability of the variant compared to the one of the native protein. The $\Delta$L activity of the variant is calculated by

$$\Delta L\ activity = (\Delta G\ stability)A - (\Delta G\ stability)I$$

-   wherein variant protein active and inactive conformations are denoted A, I for respectively the ligand free and ligand states, and AL, IL for the ligand free and ligand bound states.

For stability microswitch, if $\Delta$L activity of the variant is superior to $\Delta$L activity of the native protein, it means the variant exhibits an increased stability compared to the native protein when bound to the ligand. If $\Delta$L activity of the variant is inferior to $\Delta$L activity of the native protein, it means the variant exhibits a decreased stability compared to the native protein when bound to the ligand.

According to the invention, the prediction of the activity of the variant is based on a fitness function defined by:

$$F_{variant} = \Delta L\ activity\ (stability) + \Delta C\ activity\ (coupling)$$

The stability and coupling components can be calculated and studied independently because they correspond to two distinct allosteric properties. However, it is advantageous to use both stability and coupling component to fully describe any allosteric molecular system.

In an embodiment, the variation of allosteric coupling of said variant is chosen among :

- a) an increased constitutive activity for the active ligand free conformation A versus the inactive ligand free conformation I;

- b) an enhanced signaling response for the active ligand bound conformation AL versus the inactive ligand bound conformation IL.

Advantages of situation a (above):

- 1) should ease the structural, biophysical and pharmacological characterization of protein active states that are usually transient and unstable, on other words very difficult to isolate and characterize, for instance membrane receptor;

- 2) should facilitate the screening and identification of strong inhibitors that downregulated protein activities, for instance membrane receptor. Pharmaceutical industries usually seek such compounds using native protein. However, because native protein have very low intrinsic activities, inhibitors are very hard to find using such approach. Constitutively activated protein variants would provide well behaved targets with a large window of detection of potent inhibitors.

Advantages of situation b (above):

- 1) new classes of biosensors for synthetic biology, drug detection can be designed to virtually any ligand that has a propensity to bind to the protein, for instance membrane receptor;

- 2) new classes of protein therapeutics can be designed that enhance the sensitivity to a particular stimulus (e.g. for instance membrane receptor such as a dopamine receptor with enhanced sensitivity to dopamine could be used in gene therapy application for Parkinson disease patients and enable better motor control despite lower levels of dopamine in the brain);

- 3) new classes of protein therapeutics can be designed that redirect a particular extracellular signal to a intracellular activities that enhance immune responses (e.g. for instance membrane receptor such as a chimeric receptor can be designed that redirects an immune-suppressive signal from the tumor microenvironment into an activation signal inside a T-cell for enhancing cancer immunotherapies).

In one embodiment, said microswitch is identified by molecular dynamic simulations, for instance long time scale molecular dynamics. Molecular dynamic simulations are for instance described in Battacharya et al, Biophysical Journal, July 2014, 107(2), 422-434. As described in Battacharya *et al,* pairs of residues with high dynamic correlations or Mutual Information located on either binding sites (i.e. extracellular and intracellular) of the receptor are identified from the simulation trajectories. Pathways linking these sites through non-covalent interactions with other residues are identified that maximize the mutual information between all the connected residues in the pathway. For instance, allosteric microswitches are defined as the residues involved in multiple pathways.

Alternatively, said microswitch can be identified from the structures of a receptor or protein in distinct signaling states. Usually, a microswitch will change conformation when the receptor or protein switches between functional states (Zhou et al., elife 2019; 8:e50279). Additionally, a microswitch can be identified through sequence covariation analysis since it is usually functionally coupled to at least another microswitch in the receptor or protein structure (Sun et al., PNAS 2016 113(13):3539-44).

In an embodiment, the 3D structures of the native protein is generated by homology modeling based on at least one homolog protein. Thus, the present invention can be applied to any protein with available homolog protein. The present invention is not limited to protein for which 3D data structure are available. For instance, so far, less than 5% of all GPCRs have been structurally characterized. Using homology modeling enables to expand the structural coverage and provide reliable structural models for close to 40% of all GPCRs.

In one embodiment, said in silico mutations process is based on random mutagenesis using genetic algorithm. The Genetic algorithm (GA) evolves an initial population of protein sequences to optimize its fitness over multiple generations using two genetic operators: point mutagenesis and cross over recombination between 2 sequences. For a given population of sequences, the GA will calculate the fitness for each sequence. Then, it will create a new population of sequences defining the subsequent generation by preferentially selecting and modifying a subset of the current sequences that have the highest fitness. The probabilities for point mutagenesis and cross over recombination by the GA are defined by the user beforehand.

In one embodiment, the protein is chosen among protein membrane receptor or soluble protein. A protein membrane receptor, i.e. membrane receptor, is defined as protein receptor embedded in a cellular membrane. A soluble protein can also bind a ligand, but the soluble protein is not embedded in the cellular membrane.

In an embodiment, said receptor regulated by allosteric pathway is chosen among GPCR. The method could be applied to other families of multi-pass and single-pass receptors including Cytokine, Tyrosine kinases but also transporters, channels.

In one embodiment, the method further comprises a validation step for testing, preferably *in vitro* and/or *in cellulo* testing, preferably *in vitro*, preferably *in cellulo*, the predicted activity of the selected variant. It allows to improve the success rate of computational techniques. For instance, the present invention achieves about 80% success rate. A validation step helps to identify and rank the designed protein based on their measured activities. The validation step could also help increasing the success rate by incorporating the result of the validation step to proofread or improve the method according to the present invention, for instance the generation of *in silico* mutation.

The feedback from the experiments (i.e. failures, successes) is advantageous as it enables to optimize the computational approach and parameters guiding the calculations.

In an embodiment, the method is arranged for generating a variant of protein with an improved parameter for the ligand compared to the one of said ligand with the native protein, said parameter being chosen among sensitivity (defined as the capacity of the ligand to activate the protein), selectivity, affinity, in particular sensitivity.

In one embodiment, the method is arranged for generating a variant of protein designed for interacting with a ligand distinct from or identical to the ligand interacting with the native protein. The present invention enables the design of protein (for instance receptor or biosensor) with fine-tuned properties to a large diversity of ligands without having to design protein (for instance receptor or biosensor) from scratch which is very challenging. By rationally reprogramming the function of existing protein (for instance receptor or biosensor) through a minimal number of mutations, the present invention achieves high success rate and efficiency and can be applied to design protein (for instance receptor or biosensor) for a large variety of ligands and signals.

The invention also concerns a computer implemented program comprising instructions which, when the program is executed by a computer, cause the computer to carry out the method according to the present invention.

Also described herein is a data processing apparatus comprising means for carrying out the method according to the present invention.

The particular advantages of the data processing apparatus and of the computer implemented program are similar to the ones of the method of the invention and will thus not be repeated here.

Also described herein is a variant of a protein based on a native protein (hereafter "a protein"), or an active fragment or analog thereof, which protein sequence has been determined, designed, obtained, or is obtainable, by the method according to the present invention, wherein the sequence of said protein, or active fragment or analog thereof, comprises at least one mutation in the microswitch region.

Alternatively described herein is a protein, or an active fragment or analog thereof, obtained by any method, wherein the sequence of said protein, or active fragment or analog thereof, comprises at least one mutation in the microswitch region.

Preferably, the sequence of the protein of the invention, active fragment or analog thereof, comprises at least one mutation in the microswitch region. Preferably, the sequence of the protein of the invention, active fragment or analog thereof, comprises at least one, for example, 1, 2, 3, 4, 5, etc. mutation(s) in the microswitch region.

As used herein, the terms "protein" or "polypeptide" are used interchangeably and are intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "protein," "amino acid chain," or any other term used to refer to a chain or chains of two or more amino acids, are included within the definition of "polypeptide," and the term "polypeptide" may be used instead of, or interchangeably with any of these terms. The terms "polypeptide" or "protein" are also intended to refer to the products of post-expression modifications of the polypeptide, including without limitation glycosylation, acetylation, phosphorylation, amidation, derivatization by known protecting/blocking groups, proteolytic cleavage, or modification by non- naturally occurring amino acids as disclosed herein.

The "protein" or "polypeptide" refers to any protein or polypeptide with available homolog protein. The present invention is thus not limited to protein for which 3D data structure are available

In one aspect, the protein is selected from the group comprising multi-pass and single-pass receptors including Cytokine, Tyrosine kinases but also transporters, channels, G protein coupled receptors (GPCR), i.e. rhodopsin (family A), secretin (family B), glutamate (family C), adhesion and Frizzled/Taste2.

In case the protein of the invention is a dopamine receptor, more preferably a dopamine D2 receptor, then the sequence of said D2 dopamine receptor will comprise at least one mutation in one or more of the following amino acid position(s) (when referring to the wild-type sequence): 76, 90, 122, 205, 209, 374, 378, 379, 381, 382, 385, 421, 426, and 429. Preferably, the at least one mutation is comprised in one or more of the following amino acid position(s): 205, 374, 378, 381, and 421. More preferably, all five amino acid positions: 205, 374, 378, 381, and 421 are mutated.

In an aspect, the ligand sensing and/or signaling response is modified when compared to the native sequence of said protein.

In one aspect, the ligand sensing and/or signaling response is enhanced or decreased when compared to the native sequence of said protein.

Also described herein is a polynucleotide encoding a protein, or an active fragment or analog thereof, according to the present invention.

A "fragment" of a protein, peptide or polypeptide of the invention refers to a sequence containing less amino acids in length than the protein, peptide or polypeptide of the invention. This sequence can be used as long as it exhibits the same properties, i.e. is biologically active, as the native sequence from which it derives.

The term "analog" refers to a protein, peptide or polypeptide of the invention having an amino acid sequence that differ to some extent from a native sequence peptide, that is an amino acid sequence that varies from the native sequence by amino acid substitutions, whereby one or more amino acids are substituted by another with same characteristics and conformational roles. The amino acid sequence analogs possess substitutions, deletions, and/or insertions at certain positions within the amino acid sequence of the native amino acid sequence. Substitutions can be conservative or non-conservative. Conservative amino acid substitutions are known in the art, and include amino acid substitutions in which one amino acid having certain physical and/or chemical properties is exchanged for another amino acid that has the same chemical or physical properties. For instance, the conservative amino acid substitution may be an acidic amino acid substituted for another acidic amino acid (e.g., Asp or Glu), an amino acid with a nonpolar side chain substituted for another amino acid with a nonpolar side chain (e.g., Ala, Gly, Val, Ile, Leu, Met, Phe, Pro, Trp, Val, etc.), a basic amino acid substituted for another basic amino acid (Lys, Arg, etc.), an amino acid with a polar side chain substituted for another amino acid with a polar side chain (Asn, Cys, Gln, Ser, Thr, Tyr, etc.), etc. Preferably, the substitutions, deletions, and/or insertions at certain positions within the amino acid sequence of the amino acid sequence occur in a region that is different from the microswitch region.

In general, the sequences of such analogs will have a high degree of sequence homology to the reference (native) sequence, e.g., sequence homology of more than 50%, generally more than 60%-70%, even more particularly 80%-85% or more, such as at least 90%-95% or more, when the two sequences are aligned.

Both the analog and fragment of the protein of the present disclosure can include synthetic, non-standard and/or naturally-occurring amino acid sequences (including D-forms and/or retro-inverso isomers) derivable from the naturally occurring amino acid sequence of the protein of the invention. By way of example, the replacement amino acid may be a basic non-standard amino acid, (e.g. L-Ornithine, L-2-amino-3-guanidinopropionic acid, or D-isomers of Lysine, Arginine and Ornithine). Methods for introducing non-standard amino acids into proteins are known in the art, and include recombinant protein synthesis using E. coli auxotrophic expression hosts.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins.

Also described herein are one or more polynucleotide(s) encoding a protein, or an active fragment or analog thereof, of the invention.

Also contemplated herein is a gene delivery vector or expression vector or gene therapy vector, preferably in the form of a plasmid or a vector, which comprises one or more polynucleotide(s) encoding a protein, or an active fragment or analog thereof of the invention.

As used herein, a "vector" is capable of transferring nucleic acid sequences to target cells (e.g., viral vectors, non-viral vectors, particulate carriers, and liposomes). The terms "expression vector", "gene delivery vector" and "gene therapy vector" refer to any vector that is effective to incorporate and express one or more nucleic acid(s), in a cell, preferably under the regulation of a promoter. A cloning or expression vector may comprise additional elements, for example, regulatory and/or post-transcriptional regulatory elements in addition to a promoter, as well as tags at the C- or N- terminus of the nucleic acid to be expressed (e.g. HA signal sequence(s), His tag(s) or flag(s)). The promoter can be inducible and/or cell type-specific for specific expression in a tissue (e.g. neurons such as dopaminergic or serotoninergic neurons, glial cells, ...).

Suitable vectors include derivatives of SV40 and known bacterial plasmids, e. g., E. coli plasmids col EI, pCRI, pBR322, pLive, pMB9 and their derivatives, plasmids such as RP4; phage DNAs, e. g., the numerous derivatives of phage X, e. g., NM989, and other phage DNA, e. g., MI 3 and filamentous single stranded phage DNA; yeast plasmids such as the $2\mu$ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. Various viral vectors are used for expressing or delivering nucleic acid to cells in vitro or in vivo. Non-limiting examples are vectors based on Herpes Viruses, Baculovirus (e.g. pFastBac expression vector) Pox- viruses, Adeno-associated virus, Lentivirus, and others. In principle, all of them are suited to deliver the expression cassette comprising one or more polynucleotide(s) encoding the peptides of the invention, variants or fragments thereof and a promoter, optionally an inducible and/or cell type-specific promoter.

Also contemplated herein is a host cell comprising a plasmid or vector of the invention or one or more nucleic acid(s) encoding the peptides of the invention, analogs or fragments thereof. The host cell can be any prokaryotic or eukaryotic cell, preferably the host cell is a eukaryotic cell, most preferably the host cell is a mammalian cell. Even more preferably, the host cell is a human neuronal or glial cell.

In one aspect, the present disclosure further provides pharmaceutical compositions comprising a therapeutically effective amount of i) a plasmid or a vector of the invention, ii) a host cell of the invention, iii) a polynucleotide encoding a protein, or an active fragment or analog thereof, of the invention, or a iii) a protein of the invention, an active fragment or analog thereof, and a pharmaceutically acceptable excipient, diluent, carrier, salt and/or additive

"Pharmaceutically acceptable diluent or carrier" means a carrier or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable diluent or carrier include starch, glucose, lactose, sucrose, sodium stearate, glycerol monostearate, talc, sodium chloride, dried skim milk, glycerol, propylene glycol, water, ethanol and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 199).

Alternatively, the pharmaceutical composition of the disclosure further comprises one or more additional therapeutic agent.

Alternatively, the vector is a gene delivery vector for site-directed mutagenesis, preferably a viral vector issued for delivering a gene editing system (CRISP, TALEN, etc...) comprising i) at least one sgRNA, or crRNA and tracrRNA, targeting the genomic sequence (target DNA) encoding the protein of interest, and ii) a structureguided endonuclease such as an RNA-guided endonuclease. Any suitable naturally occurring, or engineered, RNA-guided endonuclease can be employed as long as it is effective for specifically binding a target DNA of the invention and it may be selected from the non-limiting group comprising Cas9, Cpf1, and FEN-1. Preferably, the RNA-guided endonuclease is Cas9.

Also described herein is a method of treating and/or preventing a disease such as e.g. a neurodegenerative disease or condition, or an associated symptom, in a subject in need thereof, the method comprising administering to said subject a pharmaceutical composition of the invention. Examples of neurodegenerative diseases or conditions comprise Parkinson, Schizophrenia, Huntington, Attention Deficit and Hyperactivity Disorder, and Addiction.

In one aspect of the disclosure, the protein of the invention is selected from the group comprising from SEQ ID No. 1, SEQ ID No 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 33, or an active fragment or analog thereof, or any combination thereof.

The protein, fragment or analog thereof, of the disclosure, can be prepared by a variety of methods and techniques known in the art such as for example chemical synthesis or recombinant techniques as described in Maniatis et al. 1982, Molecular Cloning, A laboratory Manual, Cold Spring Harbor Laboratory.

The protein, fragment or analog thereof, of the disclosure, is/are preferably produced, recombinantly, in a cell expression system. A wide variety of unicellular host cells are useful in expressing the polynucleotide sequence (e.g. DNA) encoding the protein, fragment or analog thereof of this invention. These hosts may include well known eukaryotic and prokaryotic hosts, such as strains of E. coli, Pseudomonas, Bacillus, Streptomyces, fungi such as yeasts, and animal cells, such as CHO, YB/20, NSO, SP2/0, RI. 1, B-W and L-M cells, African Green Monkey kidney cells (e. g., COS 1, COS 7, BSCI, BSC40, and BMTIO), insect cells (e. g., Sf9), and human cells and plant cells in tissue culture.

Preferably, the protein of the disclosure, or active fragment or analog thereof, is characterized in that the ligand sensing and/or signaling response is modified when compared to the native sequence of said protein, or active fragment or analog thereof.

More preferably, the ligand sensing and/or signaling response is enhanced or decreased when compared to the native sequence of said protein, or active fragment or analog thereof. Even more preferably, the ligand sensing and/or signaling response is enhanced when compared to the native sequence of said protein, or active fragment or analog thereof, as shown in the examples and figures.

The present disclosure also contemplates an *in-vivo* or *in-vitro* method of identifying an agent that modulates the signaling response of a variant of a protein, or an active fragment or analog thereof, wherein the sequence of said protein variant, or active fragment thereof, comprises at least one mutation in the microswitch region, the method comprising:

(1) providing a cell, or a part thereof, expressing a variant of a protein, or an active fragment or analog thereof;

(2) contacting the cell with a test agent;

(3) determining the level of activity of said variant of a protein, or an active fragment or analog thereof;

(4) comparing the level of activity with a control sample not contacted by the test agent; and

(5) selecting a test agent that decreases or enhances the signaling response of a variant of a protein, or an active fragment or analog thereof.

The embodiments described for the method also apply to the data processing and computer implemented program according to the present invention mutatis mutandis.

Further particular advantages and features of the invention will become more apparent from the following non-limitative description of at least one embodiment of the invention.

## Examples

The present examples are intended to illustrate the invention in a non-limitative manner since any feature of an embodiment may be combined with any other feature of a different embodiment in an advantageous manner.

In the following examples, the present invention is applied on GPCR to generate variants of GPCR but the invention is not limited to this class of receptor. The present invention can also be applied to other class of protein receptor.

GPCRs are prime examples of signal transduction across biological membranes. These receptors have evolved to activate several key intracellular pathways and physiological processes in response to a large diversity of extracellular stimuli. GPCR dysfunctions including misfolding, stability loss, and altered signaling activity are often associated with serious diseases. Furthermore, drugs triggering severe side-effects have been found to activate undesired signaling pathways. Hence, the mapping of receptor stability and signal transduction pathways determinants would greatly enhance the development of effective therapies and personalized medicine approaches but has remained very challenging.

In the present example, the point was to generate (in other words engineer) dopamine D2 receptor variants, i.e. proteins of the invention.

3D structures:

In the present example, the 3D structures of ligand free and ligand bound states were engineered by homology modeling.

For the ligand free 3D structures, the closest structurally characterized homologs available were used as templates to model the inactive and active state. The inactive state conformations were modeled starting from the X-ray structure of the antagonist bound dopamine D3 receptor (DRD3, PDBID: 3PBL) sharing 48% sequence identity with D2. The active state conformations were modeled from the active state GPCR structures of two distant homologs: opsin (23% sequence identity with D2) bound to Gt (PDBID: 3CAP, 3DQB) and beta-2 adrenergic receptor (26% sequence identity with D2) in complex with ligand agonist and heterotrimeric Gs (PDBID: 3SN6). Models of D2 inactive and active states were generated without bound ligand or G protein using the homology mode of RosettaMembrane.

From the above-mentioned simulations, ensembles of low energy models were clustered and centers of the most populated clusters were selected as templates for each ligand-free state in the design calculations of stability microswitches. An inactive and an active state models of D2 WT were selected therefrom.

Ligand-bound D2 models were generated by performing ligand docking simulations onto the inactive and active state ligand free D2 models obtained as described above. The ligand conformer libraries were generated using Omega (OpenEye Inc.) with default parameters from Rosetta ARLS. Ligand docking was performed using a combination of Monte Carlo moves, sidechain repacking, and gradient-based minimization with the Rosetta all-atom potential developed for protein-ligand docking. Receptor backbone and sidechain torsions along with ligand torsions, position, and orientation were optimized simultaneously.

All ligand-bound receptor models for a given state (i.e. inactive or active) were gathered together and the 5% lowest-energy models were selected and ranked based on the binding energy with the ligand. The selected ligand bound models were then clustered based on structural similarity of the bound ligand conformation. The representative model of the largest cluster was selected corresponding to the ligand bound model of the D2 receptor in a given state; i.e. ligand-bound D2 inactive and ligand-bound D2 active state conformations.

Allosteric site(s), i.e. microswitches, were defined by mapping the allosteric residues identified using Molecular Dynamics simulations performed on the homologous B2AR receptor onto the above-mentioned D2 structures.

GPCR structures are composed of 3 main regions: the extracellular ligand binding pocket, the intracellular G-protein binding domain and the "transmission" transmembrane (TM) region which connects the two binding regions and allows them to communicate. GPCRs typically switch from inactive to active state conformations upon activating agonist ligand and G-protein binding at the extracellular and intracellular domains, respectively. The structural rearrangements upon receptor activation involve large intracellular reorientations of transmembrane helices (TMH), notably TMH6 and TMH7 and smaller scale movements of individual amino-acids named microswitch across the entire TM domain.

**Example 1**

In the present example 1, the TM domain of the selected ligand free and ligand bound models were scanned to identify microswitches with novel allosteric properties at the position 205. Usually, larger number of positions are designed simultaneously.

In the present example, the following amino acids were identified as microswitch:

- Threonine 205 that is in close proximity to other microswitches including Phenylalanine 202, Phenylalanine 382, Isoleucine 122.

Generation of variants

Random mutagenesis using all 20 possible amino acids at position 205 and search+selection using a Genetic algorithm were performed to optimize the following fitness function :

$$F = W_{stability} \left( \Delta E_s^A - \Delta E_s^I \right) + W_{allostery} \left( \Delta E_c^A - \Delta E_c^I \right) = W_{stability} \left( \Delta \Delta E_s \right) + W_{allostery} \left( \Delta \Delta E_c \right).$$

When the microswitch is selected for stability in the active state (A) only:
$W_{stability} = 1$; $W_{allostery} = 0$; the microswitch with the highest fitness was Isoleucine 205.

When the microswitch is selected for higher sensitivity to ligand binding only,
$W_{stability} = 0$; $W_{allostery} = 1$; the microswitch with the highest fitness was Methionine 205.

Computing of the score

T2051: $\Delta G$-stability or $\Delta \Delta E_s$ = 5.4; $\Delta G$-coupling or $\Delta \Delta E_c$ = 0.1

T205M: $\Delta G$-stability or $\Delta \Delta E_s$ = 0.1; $\Delta G$-coupling or $\Delta \Delta E_c$ = 0.8

Prediction of the activity and in vitro confirmation of the predicted activity

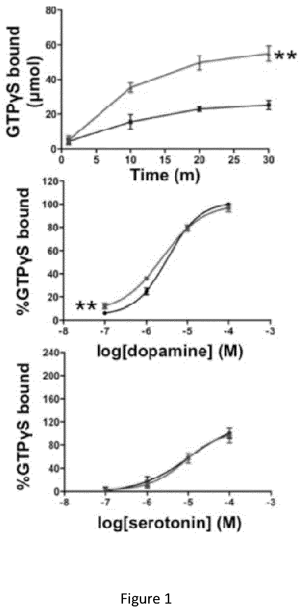

Regarding the T205I mutation, since $\Delta G$-stability/$\Delta \Delta E_s$ >0, the protein obtained by the method of the invention should have an increased constitutive activity compared to the native receptor. This prediction was confirmed by in vitro testing where this increased of constitutive activity was observed experimentally (top panel of Figure 1. Figure 1 shows the *in vitro* measurements of D2 activation. top. constitutive (time course); bottom. Agonist induced (dose response) as percent of maximal dopamine induced D2 WT activity. ** two-sided unpaired t-test: p < 0.05.). Therefore, the present invention allows to generate a D2 receptor stabilized in specific states, here in state A.

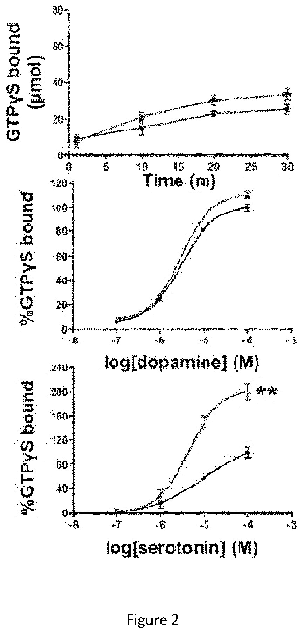

Regarding the T205M mutation, since $\Delta$G-coupling/$\Delta\Delta$Ec >0, the protein obtained by the method of the invention should have a higher sensitivity for the ligand binding (ligand = dopamine and serotonin). This prediction was confirmed by in vitro testing where this increased of sensitivity was observed experimentally (bottom panel of Figure 2. Figure 2 shows the *in vitro* measurements of D2 activation. top. constitutive (time course); bottom. Agonist induced (dose response) as percent of maximal dopamine induced D2 WT activity. ** two-sided unpaired t-test: p < 0.05.). Therefore, the present invention allows to generate a D2 receptor with an increased sensitivity for both dopamine and serotonin ligands.

### Example 2

**Computational modeling of DRD2 active and inactive states.** The inactive and active state structures of DRD2 WT were modeled from the close homolog inactive state structure of DRD3 (3PBL) and the more distant homolog active state structure of $\beta_2$AR, respectively, using the software iPHoLD (Draper-Joyce, C. J. et al. Structure of the adenosinebound human adenosine A1 receptor-Gi complex. Nature 558, 559-563 (2018)). Low energy representative models of the DRD2 WT were selected for design calculations, as previously described.

**Computational design of residue microswitches.** The TM region of DRD2 was subjected to complete multi-state design using a fitness function selecting residue microswitches stabilizing the active state structure while decreasing the allosteric coupling between the extracellular ligand binding site and the inactive state conformation of the intracellular G protein binding site. Conformational stability was assessed using an all-atom energy function developed for membrane protein modeling and design. Allosteric couplings were approximated by the correlated dynamic fluctuations between allosteric residues using normal mode calculations (Danev, R. & Baumeister, W. Cryo-EM single particle analysis with the Volta phase plate. Elife 5, 439 (2016)).

**Computational *de novo* design of minimal intracellular loop 3.** The kinematic loop design protocol of Rosetta was performed to *de novo* design short loop structures and sequences connecting the cytoplasmic ends of TM5 and TM6. Loop structures and sequences were selected to maximize stability and optimize conformation for $G_{\alpha i}$ binding. Loop lengths of three to nine amino acids were tested and the predicted models were clustered, selected by energy and structurally analyzed for distortions of the TM5/6 helical tips. Several designed loops of five to seven residues adopted stable helical structures enabling proper $G_{\alpha i}$ binding. Selected sequences from these loops with further refined with RosettaMembrane on active state DRD2 models onto which a peptide corresponding to the $G_{\alpha i}$ C-terminal $\alpha$5 helix was docked. The final selected 7 residue long designed ICL3 was predicted to retain native receptor-G protein TM contacts while stabilizing the DRD2 active conformation.

**Mutagenesis and expression of receptors in HEK293 cells for stability assays.** The designed SEQ ID No. 33 contains 5 mutations: T205$^{5.54}$I, M374$^{6.36}$L, V378$^{6.40}$Y, V381$^{6.43}$L and V421$^{7.48}$I and a designed minimal ICL3 where residues 222-360 at ICL3 of the WT DRD2 (sequence #22) were replaced with residues LVNTN, as designed in the optimized 7-residue ICL3 described above (with one wild-type amino acid flanking each side of the insertion). The resulting construct was obtained by Quickchange PCR mutagenesis (Stratagene) performed on the HA-tagged human long isoform DRD2 gene (coding for the sequence #22) in the pcDNA3.1(+) vector. Sequenced mutant plasmids were transiently transfected using lipofectamine (Invitrogen) into HEK293T cells for DRD2 as described (Chen, K.-Y. M., Keri, D. & Barth, P. Computational design of G Protein-Coupled Receptor allosteric signal transductions. Nat. Chem. Biol. 16, 77-86 (2020).). Briefly, 5 x 10$^6$ cells were plated on 10 cm tissue culture plates and grown overnight. This was followed by transfection with Lipofectamine 2000 (Invitrogen) and 2 $\mu$g of DNA per plate. After 24 hours, the cells were washed with PBS and grown in standard growth medium (DMEM supplemented with L-glutamine (2 mM), penicillin (100 mg/mL), streptomycin (100 mg/mL), and fetal bovine serum (10%)) for an additional 24 hours prior to harvesting.

**Membrane preparation and receptor purification.** Membranes were prepared from transfected cells using sucrose gradient centrifugation as previously described (Chen et al., Nat Chem Biol 2019; doi: 10.1038/s41589-019-0407-2). Briefly, cells from 10cm plates were collected by cell scraper with PBS solution. Cells were pelleted and resuspended in cold hypotonic buffer (1mM Tris-HCL, pH 6.8, 10mM EDTA, protease inhibitor cocktail). Cells were forced through a 26-gauge needle three times. The cell lysate was layered onto a 38% sucrose solution in buffer A (150mM NaCl, 1mM MgCl, 10mM EDTA, 20mM Tris-HCl, pH 6.8, protease inhibitor cocktail) in SW-28 ultracentrifuge tubes. Cells were centrifuged at 15,000 rpm at 4°C for 20 minutes, followed by collection of the interface band with an 18-gauge needle. The collected solution was transferred to Ti-45 ultracentrifuge tubes and the volume brought up to 50mL with buffer A. The sample was spun at 40,000 rpm at 4°C for 30 minutes. The membrane pellets from each 10cm plate was resuspended in 0.5mL buffer A and stored at -80°C in 100uL aliquots.

Receptor variants were partially purified from thawed membrane preparations immediately prior to assaying via anti-HA agarose beads. Membrane preparations were solubilized with 1% n-dodecylmaltoside (DDM) for one hour at 4°C, and loaded onto anti-HA agarose beads (Pierce/Thermo Scientific) for one hour at 4°C. The beads were washed with TBS with 0.1% DDM wash buffer three times and HA-tagged receptor variants were eluted with HA peptide (1mg/mL in TBS with 0.1% DDM).

**Expression and purification of G proteins.** Gi2 subunits were cloned into pFastbacl (Invitrogen) followed by transformation into DH10$\alpha$ cells. Recombinant bacmid DNA was isolated and transfected in Sf9 insect cells with Cellfectin II (Invitrogen). The transfected cells were grown at 28°C for 72 hours followed by centrifugation in 15mL Falcon tubes to pellet cell debris. The supernatant was saved as P1 viral stock. Virus was amplified by infecting Sf9 cells with P1 viral stock solution at a 2-fold multiplicity-of-infection and cultured for 72 hours prior to harvesting. This amplification process was repeated to generate a high-titer P3, which was used for infection and protein expression. P3 stock was used to infect Sf9 cells at an MOI of 4 and harvested 48 hours post infection. Cells were washed three times in ice-cold PBS and resuspended in homogenization buffer (10mM Tris-HCl, 25mM NaCl, 10mM MgCl$_2$, 1mM EGTA, 1mM DTT, protease inhibitor cocktail, 10uM GDP, pH 8.0). Gi2 was purified and reconstituted as described previously (Chen et al., Nat Chem Biol 2019; doi: 10.1038/s41589-019-0407-2).

**G protein activation assays.** D2DR variants were assayed for their ability to induce guanine nucleotide exchange by the G protein Gi2. To measure constitutive activity the reaction mixture consisted of 4uM Gi2, 20uM of [$^{35}$S]-GTP$\gamma$S mix, 50mM Tris-HCl, pH 7.2, 100mM NaCl, 4mM MgCl$_2$, 1mM dithiothreitol. Receptor concentrations were ~10nM per sample. Reactions were started by adding 150uL partially purified receptor samples to 300uL of reaction mixture and incubating on ice over a period of time from 0-30 minutes for timecourse measurements. To measure ligand induced D2DR activities, increasing amounts of dopamine or spiperone were added to the reaction mixtures. Maximal ligand efficacies were obtained with final ligand concentrations of 20uM and 1uM for dopamine and spiperone, respectively. Reactions were stopped by filter binding onto Millipore nitrocellulose filters or Whatman fiberglass filters pretreated with polyethylenimine. Filters were washed three times with ice-cold TBS prior to incubation with scintillation fluid. Radioactivity counts were measured on a Beckman LS6000 scintillation counter. Statistical significance of differences in constitutive or ligand-induced activities was assessed by student t-tests.

**Ligand binding affinity.** To determine the binding affinities of dopamine and spiperone to D2DR variants, anti-HA agarose affinity purified receptor samples prepared as described above were titrated with [$^3$H]-dopamine or [$^3$H]-spiperone in a total volume of 150uL per sample, incubated for 1 hour on ice followed by loading onto Millipore nitrocellulose filters or Whatman fiberglass filters pretreated with polyethylenimine and washed for scintillation counting as mentioned above. For competition binding, receptor samples were pre-incubated with competing cold ligand for 30min on ice prior to adding saturating amount of radiolabeled hot ligand (1.2uM [$^3$H]-dopamine or 10nM [$^3$H]-spiperone). Competition binding and apparent thermostability curves were fitted using GraphPad Prism software and student t-tests were performed to assess statistical significance.

**Apparent agonist-bound D2DR stability.** Apparent stability of agonist-bound D2DR variants was determined by measuring either receptor binding to agonist (apparent meting temperature) or receptor activities (apparent receptor half-life) as a function of temperature. To measure apparent melting temperature, HA agarose affinity purified receptor samples prepared as described above were first pre-incubated at increasing temperatures for 30 min. The fraction of receptor binding dopamine was then assessed by radioligand binding assay described above. To measure apparent receptor half-life, purified receptor samples were pre-incubated at 37°C over a time period from 0-60 minutes prior to addition to the reaction mixture containing Gi, [$^{35}$S]-GTP$\gamma$S and 20uM dopamine as described above. Apparent melting temperatures and active state half-life curves were fitted using GraphPad Prism software and analyzed for statistical significance by student t-tests.

## Example 3

The Inventors computationally designed and stabilized the intracellular loop 3 of rhodopsin in a conformation that activates the downstream effector transducing in accordance with the method of the invention. Two sets of mutations located between transmembrane helical (TMH) 6 and 7 (i.e. M257Y on TMH6 and I305A on TMH7) and in intracellular loop 3 (ICL3) (K231E.E2321.T251 R) predicted to shift the receptor equilibrium toward the active state were generated.

As predicted, while both TMHs 6/7 mutants displayed much larger constitutive activities than WT opsin, the rate of transducin activation of M257Y.I305A was higher than that of M257Y alone (data not shown, manuscript in preparation).

The ICL3 triple mutant (K231E.E232I.T251R) displayed a dramatically increased constitutive activity compared to WT reaching higher than 40% of the light-induced maximal activity and an increased apparent stability (data not shown, manuscript in preparation).

The Inventors showed that receptor enhanced signaling is achieved through designed polar residues which act as functional switches by forming optimal interaction networks only in the active state loop conformation. Remarkably, designed opsins display increased conformational stability and up to 7.5 fold enhancement in transducin activation compared to wild-type opsin while retinal binding and light-induced activities remain unperturbed.

This example further demonstrates that activating microswitches and soluble loop conformations strongly modulate membrane receptor activity and suggests a novel computational protein engineering strategy to manipulate receptor mediated cellular signaling.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This is for example particularly the case regarding the different apparatuses and methods which can be used.

**Sequences**

| SEQ ID No. | Name | Name / Sequence |
|---|---|---|
| 1 | C385L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI<br><br>YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIILWLPFFIT HILNIHCDCNIPPVLYSAFTWLGYVNSAVN PIIYTTFNIEFRKAFLKILHC |
| 2 | C385V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIIVWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 3 | F202M | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPMIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |
| 4 | F382I-C385L | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVIIILWLPFFIT<br><br>HILNIHCDCNIPPVLYSAFTWLGYVNSAVN<br>PIIYTTFNIEFRKAFLKILHC |
| 5 | F382I | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVIIICWLPFFIT<br>HILNIHCDCNIPPVLYSAFTWLGYVNSAVN<br>PIIYTTFNIEFRKAFLKILHC |

(continued)

| SEQ ID No. | Name | Name / Sequence |
|---|---|---|
| 6 | F382Y | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVYIICWLPFFIT<br>HILNIHCDCNIPPVLYSAFTWLGYVNSAVN<br>PIIYTTFNIEFRKAFLKILH |
| 7 | I122F | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASF<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |
| 8 | I122L | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASL<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |

(continued)

| SEQ ID No. | Name | Name / Sequence |
|---|---|---|
| 9 | I122T | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTAST LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 10 | L379I_C 385L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVFIILWLPFFIT HILNIHCDCNIPPVLYSAFTWLGYVNSAVN PIIYTTFNIEFRKAFLKILHC |
| 11 | L379I_F 382I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVIIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 12 | L379I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVFIICWLPFFIT HILNIHCDCNIPPVLYSAFTWLGYVNSAVN PIIYTTFNIEFRKAFLKILHC |
| 13 | L7 6M | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSMAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 14 | M90V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVVPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 15 | D2_R1 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIS HILNIHCDCNIPPVGYSAWTWLGYVNSAVN PIIYTTFNIEFRKAFLKILHC |
| 16 | R1+R2+R 3+R4 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLYVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPLIVIILVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQLLAIYLGLFLICWLPFFIS HILNIHCDCNIPPVGYSAWTWLGYVNSAIN PIIFATFNIEFRKAFLKILHC |
| 17 | D2_R2 | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPLIVIILVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFLICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 18 | R2+R3 | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br><br>TVMISIVWVLSFTISCPLLFGLNNADQNEC<br>IIANPAFVVYSSIVSFYVPLIVIILVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQLLAIYLGLFLICWLPFFIT<br>HILNIHCDCNIPPVLYSAFTWLGYVNSAIN<br>PIIYTTFNIEFRKAFLKILHC |
| 19 | R2+R3+R 4 | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLYVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPLIVIILVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQLLAIYLGLFLICWLPFFIT<br>HILNIHCDCNIPPVLYSAFTWLGYVNSAIN<br>PIIFATFNIEFRKAFLKILHC |
| 20 | D2_R3 | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVILLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQLLAIYLGLFIICWLPFFIT<br>HILNIHCDCNIPPVLYSAFTWLGYVNSAIN<br>PIIYTTFNIEFRKAFLKILHC |

(continued)

| SEQ ID No. | Name | Name / Sequence |
|---|---|---|
| 21 | D2_R4 | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLYVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVTLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br><br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIFATFNIEFRKAFLKILHC |
| 22 | T205I | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVILLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |
| 23 | T205L | MYPYDVPDYAYPYDVPDYAYPYDVPDYADD<br>PLNLSWYDDDLERQNWSRPFNGSDGKADRP<br>HYNYYATLLTLLIAVIVFGNVLVCMAVSRE<br>KALQTTTNYLIVSLAVADLLVATLVMPWVV<br>YLEVVGEWKFSRIHCDIFVTLDVMMCTASI<br>LNLCAISIDRYTAVAMPMLYNTRYSSKRRV<br>TVMISIVWVLSFTISCPLLFGLNNADQNEC<br>IIANPAFVVYSSIVSFYVPFIVLLLVYIKI<br>YIVLRRRKRVNTKRSSRAFRAHLRAPLKG<br>NCTHPEDMKLCTVIMKSNGSFPVNRRRVEA<br>ARRAQELEMEMLSSTSPPERTRYSPIPPSH<br>HQLTLPDPSHHGLHSTPDSPAKPEKNGHAK<br>DHPKIAKIFEIQTMPNGKTRTSLKTMSRRK<br>LSQQKEKKATQMLAIVLGVFIICWLPFFIT<br>HILNIHCDCNIPPVLYSAFTWLGYVNSAVN<br>PIIYTTFNIEFRKAFLKILHC |

(continued)

| SEQ ID No. | Name | Name / Sequence |
|---|---|---|
| 24 | T205M_C 385L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIILWLPFFIT HILNIHCDCNIPPVLYSAFTWLGYVNSAVN PIIYTTFNIEFRKAFLKILHC |
| 25 | T205M_C 385V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIIVWLPFFIT HILNIHCDCNIPPVLYSAFTWLGYVNSAVN PIIYTTFNIEFRKAFLKILHC |
| 26 | T205M_F 382I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIIVWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 27 | T205M_L 379I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVIGVFIICWLPFFIT HILNIHCDCNIPPVLYSAFTWLGYVNSAVN PIIYTTFNIEFRKAFLKILHC |
| 28 | T205V | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV<br><br>TVMISIVWVLSFTISCPLLFGLNNADQNEC IIANPAFVVYSSIVSFYVPFIVVLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 29 | Y209L | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVLIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 30 | Y426F | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIFTTFNIEFRKAFLKILHC |
| 31 | T205M | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVMLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK<br><br>DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |
| 32 | D2WT | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRKRVNTKRSSRAFRAHLRAPLKG NCTHPEDMKLCTVIMKSNGSFPVNRRRVEA ARRAQELEMEMLSSTSPPERTRYSPIPPSH HQLTLPDPSHHGLHSTPDSPAKPEKNGHAK DHPKIAKIFEIQTMPNGKTRTSLKTMSRRK LSQQKEKKATQMLAIVLGVFIICWLPFFIT HILNIHCDCNIPPVLYSAFTWLGYVNSAVN PIIYTTFNIEFRKAFLKILHC |

(continued)

| SEQ ID No. | Name | Name / Sequence |
|---|---|---|
| 33 | T205I_ M374L_ V378Y_ V381L_ V421I | MYPYDVPDYAYPYDVPDYAYPYDVPDYADD PLNLSWYDDDLERQNWSRPFNGSDGKADRP HYNYYATLLTLLIAVIVFGNVLVCMAVSRE KALQTTTNYLIVSLAVADLLVATLVMPWVV YLEVVGEWKFSRIHCDIFVTLDVMMCTASI LNLCAISIDRYTAVAMPMLYNTRYSSKRRV TVMISIVWVLSFTISCPLLFGLNNADQNEC IIANPAFVVYSSIVSFYVPFIVTLLVYIKI YIVLRRRRKLVNTNRKLSQQKEKKATQLLA IYLGLFIICWLPFFITHILNIHCDCNPPVL YSAFTWLGYVNSAINPIIYTTFNIEFRKAF LKILHC |

## Claims

1. Computer implemented method for generating variants of a protein based on a native protein regulated by allosteric pathway, the method comprising:

   - i) providing 3D structures of the native protein, said 3D structures comprising protein active conformation and inactive conformation for both ligand free and ligand bound states;
   - ii) identifying at least one pair of coupled allosteric sites within the amino acid sequence of the native protein, each allosteric site being named microswitch, each microswitch consisting in one amino acid involved in regulating a signal transduced by the native protein,
   - iii) generating *in silico* mutations of said identified microswitch to generate a pool of variants whose amino acid sequences comprise at least one mutation compared to the native sequence,
   - iv) computing at least one score reflecting

     - iv)a) a variation in allosteric coupling for each variant compared to the allosteric coupling of the native protein, said variation being named ΔG-coupling; and/or
     - iv)b) a variation in the relative stability of each conformation for each variant, said variation being named ΔG-stability;

   - v) predicting the activity of each variant compared to the native protein based on the computed score ΔG-coupling or ΔG-stability, namely

     - v)a) a change in ligand-induced activity of the variant compared to the native protein named ΔL activity, said change being calculated by

$$\Delta L \text{ activity} = (\Delta G \text{ coupling})AL - (\Delta G \text{ coupling})IL;$$

     and/or
     - v)b) a change in ligand free activity of the variant compared to the native protein named ΔC activity, said change being calculated by

$$\Delta C \text{ activity} = (\Delta G \text{ stability})A - (\Delta G \text{ stability})I$$

     wherein for the ligand free state conformations, variant protein active and inactive conformations are denoted respectively A and I;
     whereas for the ligand bound state conformations, variant protein active and inactive conformations are denoted respectively AL and IL;
     and wherein the prediction of the activity of the variant is based on a fitness function defined by:

$$F_{variant} = \Delta L \text{ activity (stability)} + \Delta C \text{ activity (coupling)}$$

- vi) *in vitro* testing the predicted activity of a selected variant in a validation step;
wherein the method is arranged for generating a variant of protein with an improved parameter for the ligand compared to the one of said ligand with the native protein, said parameter being chosen among selectivity, specificity, affinity, preferably sensitivity.

2. Method according to claim 1, wherein ΔG-coupling is calculated from dynamics correlations between identified microswitches using an elastic model of the protein.

3. Method according to any one of claims 1 to 2, wherein ΔG-stability is calculated from the sum of all interactions between the amino acids of the protein in a specific conformation and in absence of ligand.

4. Method according to any one of claims 1 to 3, wherein the variation of allosteric coupling of said variant is chosen among :

- a) an increased constitutive activity for the active ligand free conformation A versus the inactive ligand free conformation I;
- b) an enhanced signaling response for the active ligand bound conformation AL versus the inactive ligand bound conformation IL.

5. Method according to any one of claims 1 to 4, wherein the protein is chosen among membrane receptor, soluble protein, for instance GPCR, cytokine, tyrosine kinases, transporters, channels.

6. Method according to any one of claims 1 to 5, wherein the method is arranged for generating a variant of protein designed for interacting with a ligand distinct from or identical to the ligand interacting with the native protein.

7. Computer implemented program comprising instructions which, when the program is executed by a computer, cause the computer to carry out the method of any one of claims 1 to 6.

**Patentansprüche**

1. Computerimplementiertes Verfahren zum Erzeugen von Varianten eines Proteins basierend auf einem nativen Protein, das durch den allosterischen Weg reguliert wird, wobei das Verfahren Folgendes umfasst:

- i) Bereitstellen von 3D-Strukturen des nativen Proteins, wobei die 3D-Strukturen die aktive Konformation und die inaktive Konfirmation des Proteins sowohl für den ligandenfreien als auch den ligandengebundenen Zustand umfassen;
- ii) Identifizieren mindestens eines Paares gekoppelter allosterischer Stellen innerhalb der Aminosäuresequenz des nativen Proteins, wobei jede allosterische Stelle als Mikroschalter bezeichnet wird und jeder Mikroschalter aus einer Aminosäure besteht, die an der Regulierung eines Signals beteiligt ist, das von dem nativen Protein übertragen wird,
- iii) Erzeugen von In-silico-Mutationen des identifizierten Mikroschalters, um einen Pool von Varianten zu erzeugen, deren Aminosäuresequenzen im Vergleich zu der nativen Sequenz mindestens eine Mutation umfassen,
- iv) Berechnen mindestens eines Scores, der Folgendes widerspiegelt:

- iv)a) eine Variation der allosterischen Kopplung für jede Variante im Vergleich zur allosterischen Kopplung des nativen Proteins widerspiegelt, die als ΔG-Kopplung bezeichnet wird; und/oder
- iv) b) eine Variation der relativen Stabilität jeder Konformation für jede Variante, wobei die Variation als ΔG-Stabilität bezeichnet wird;

- v) Vorhersagen der Aktivität jeder Variante im Vergleich zu dem nativen Protein basierend auf dem Score ΔG-Kopplung oder ΔG-Stabilität, nämlich

- v)a) eine Änderung der ligandeninduzierten Aktivität der Variante im Vergleich zu dem nativen Protein, die

als $\Delta$L-Aktivität bezeichnet wird, wobei die Änderung berechnet wird durch

$$\Delta\text{L-Aktivität} = (\Delta\text{G-Kopplung})\text{AL} - (\Delta\text{G-Kopplung})\text{IL};$$

und/oder

- v)a) eine Änderung der ligandenfreien Aktivität der Variante im Vergleich zu dem nativen Protein, die als $\Delta$C-Aktivität bezeichnet wird, wobei die Änderung berechnet wird durch

$$\Delta\text{C-Aktivität} = (\Delta\text{G-Stabilität})\text{A} - (\Delta\text{G-Stabilität})\text{I},$$

wobei für die Konformationen im ligandenfreien Zustand die aktive und die inaktive Konformation des Variantenproteins als A bzw. I bezeichnet werden;
wobei für die Konformationen im ligandengebundenen Zustand die aktive und die inaktive Konformation des Variantenproteins als AL bzw. IL bezeichnet werden;
und wobei die Vorhersage der Aktivität der Variante auf einer Fitnessfunktion basiert, die definiert ist durch:

$$F_{\text{Variante}} = \Delta\text{L-Aktivität (Stabilität)} + \Delta\text{C-Aktivität (Kopplung)}$$

- vi) *In-vitro*-Testen der vorhergesagten Aktivität einer ausgewählten Variante in einem Validierungsschritt;
wobei das Verfahren dazu ausgelegt ist, eine Proteinvariante mit einem verbesserten Parameter für den Liganden im Vergleich zu dem des Liganden mit dem nativen Protein zu erzeugen, wobei der Parameter aus Selektivität, Spezifität, Affinität und vorzugsweise Sensitivität ausgewählt wird.

2. Verfahren nach Anspruch 1, wobei die $\Delta$G-Kopplung aus Dynamik-Korrelationen zwischen identifizierten Mikroschaltern unter Verwendung eines elastischen Modells des Proteins berechnet wird.

3. Verfahren nach einem der Ansprüche 1 bis 2, wobei die $\Delta$G-Stabilität aus der Summe aller Wechselwirkungen zwischen den Aminosäuren des Proteins in einer spezifischen Konformationen und in Abwesenheit eines Liganden berechnet wird.

4. Verfahren nach einem der Ansprüche 1 bis 3, wobei die Variation der allosterischen Kopplung der Variante aus Folgenden ausgewählt wird:

   - a) einer erhöhten konstitutiven Aktivität für die aktive ligandenfreie Konformation A im Vergleich zu der inaktiven ligandenfreien Konformation I;
   - a) einer verstärkten Signalantwort für die aktive ligandengebundene Konformation AL im Vergleich zu der inaktiven ligandengebundenen Konformation IL.

5. Verfahren nach einem der Ansprüche 1 bis 4, wobei das Protein aus Membranrezeptoren, löslichen Proteinen, beispielsweise GPCR, Zytokinen, Tyrosinkinasen, Transportern, Kanälen ausgewählt wird.

6. Verfahren nach einem der Ansprüche 1 bis 5, wobei das Verfahren dazu ausgelegt ist, eine Proteinvariante zu erzeugen, die für die Wechselwirkung mit einem Liganden konzipiert ist, der sich von dem mit dem nativen Protein wechselwirkenden Liganden unterscheidet oder damit identisch ist.

7. Computerimplementiertes Programm, umfassend Anweisungen, die bei Ausführung des Programms durch einen Computer den Computer zum Durchführen des Verfahrens nach einem der Ansprüche 1 bis 6 veranlassen.

## Revendications

1. Procédé mis en oeuvre par ordinateur pour générer des variantes d'une protéine sur la base d'une protéine endogène régulée par voie allostérique, le procédé comprenant :

   i. fournir des structures 3D de la protéine endogène, lesdites structures 3D comprenant une conformation active et une conformation inactive de la protéine pour les états libres et liés au ligand ;

ii. identifier au moins une paire de sites allostériques couplés au sein de la séquence d'acides aminés de la protéine endogène, chaque site allostérique étant nommé micro-interrupteur, chaque micro-interrupteur étant constitué d'un acide aminé impliqué dans la régulation d'un signal transduit par la protéine endogène,

iii. générer des mutations *in silico* dudit micro-interrupteur identifié pour générer un groupe de variants dont les séquences d'acides aminés comprennent au moins une mutation par rapport à la séquence endogène,

iv. calculer au moins un score reflétant

iv)a) une variation du couplage allostérique pour chaque variant par rapport au couplage allostérique de la protéine endogène, ladite variation étant appelée couplage ΔG- ; et/ou

iv)b) une variation de la stabilité relative de chaque conformation pour chaque variant, ladite variation étant nommée ΔG-stabilité ;

v. prédire l'activité de chaque variant par rapport à la protéine endogène en fonction du score calculé de couplage ΔG ou de stabilité ΔG, à savoir

v)a) un changement dans l'activité induite par le ligand du variant par rapport à la protéine endogène appelée activité ΔL, ledit changement étant calculé par

$$\text{activité } \Delta L = (\text{couplage } \Delta G)AL - (\text{couplage } \Delta G)IL ;$$

et/ou

v)b) un changement dans l'activité sans ligand du variant par rapport à la protéine endogène appelée activité ΔC, ledit changement étant calculé par

$$\text{activité } \Delta C = (\text{stabilité } \Delta G)A - (\text{stabilité } \Delta G)I$$

dans lequel pour les conformations de l'état sans ligand, les conformations actives et inactives des protéines variantes sont désignées respectivement A et I ;

alors que pour les conformations de l'état lié au ligand, les conformations actives et inactives des protéines variantes sont désignées respectivement par AL et IL ;

et dans lequel l'activité prédite du variant est basée sur une fonction d'aptitude définie par

$$F_{variant} = \text{activité } \Delta L (\text{stabilité}) + \text{activité } \Delta C (\text{couplage})$$

vi. tester *in vitro* l'activité prédite d'un variant sélectionné dans une étape de validation ;

dans lequel le procédé est agencé pour générer un variant de protéine avec un paramètre amélioré pour le ligand par rapport à celui dudit ligand avec la protéine endogène, ledit paramètre étant choisi parmi la sélectivité, la spécificité, l'affinité, de préférence la sensibilité.

2. Procédé selon la revendication 1, dans lequel le couplage ΔG est calculé à partir des corrélations dynamiques entre les micro-interrupteurs identifiés à l'aide d'un modèle élastique de la protéine.

3. Procédé selon la revendication 1 ou 2, dans lequel la stabilité ΔG est calculé à partir de la somme de toutes les interactions entre les acides aminés de la protéine dans une conformation spécifique et en l'absence de ligand.

4. Procédé selon l'une des revendications 1 à 3, dans lequel la variation de couplage allostérique dudit variant est choisie parmi :

a) une activité constitutive accrue pour la conformation active sans ligand A par rapport à la conformation inactive sans ligand I ;

b) une réponse de signalisation accrue pour la conformation active liée au ligand AL par rapport à la conformation inactive liée au ligand IL.

5. Procédé selon l'une des revendications 1 à 4, dans lequel la protéine est choisie parmi un récepteur membranaire, une protéine soluble, par exemple GPCR, une cytokine, des tyrosine kinases, des transporteurs, des canaux.

6. Procédé selon l'une des revendications 1 à 5, dans lequel la méthode est agencé pour générer un variant de protéine conçu pour interagir avec un ligand distinct ou identique au ligand interagissant avec la protéine endogène.

7. Programme mis en oeuvre par ordinateur comprenant des instructions qui, lorsque le programme est exécuté par un ordinateur, amènent l'ordinateur à mettre en oeuvre le procédé selon l'une des revendications 1 à 6.

Figure 1

Figure 2

Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0218590 A
- WO 2010149964 A

**Non-patent literature cited in the description**

- **BATTACHARYA et al.** *Biophysical Journal*, July 2014, vol. 107 (2), 422-434
- **ZHOU et al.** *elife*, 2019, vol. 8, e50279
- **SUN et al.** *PNAS*, 2016, vol. 113 (13), 3539-44
- **MANIATIS et al.** Molecular Cloning, A laboratory Manual. Cold Spring Harbor Laboratory, 1982
- **DRAPER-JOYCE, C. J. et al.** Structure of the adenosinebound human adenosine A1 receptor-Gi complex. *Nature*, 2018, vol. 558, 559-563
- **DANEV, R.** ; **BAUMEISTER, W**. Cryo-EM single particle analysis with the Volta phase plate. *Elife*, 2016, vol. 5, 439
- **CHEN, K.-Y. M.** ; **KERI, D.** ; **BARTH, P**. Computational design of G Protein-Coupled Receptor allosteric signal transductions. *Nat. Chem. Biol.*, 2020, vol. 16, 77-86
- **CHEN et al.** *Nat Chem Biol*, 2019